# EUROPEAN PATENT SPECIFICATION

(11) **EP 4 581 041 B1**
(45) Date of publication and mention of the grant of the patent: **26.08.2026**
(21) Application number: 23809015.3
(22) Date of filing: 13.10.2023
(51) Int. Cl.: C07F 9/54, C07F 9/6574, C08G 65/10, C08G 65/26

(54) **ALKOXYLATION PROCESSES USING PHOSPHONIUM DICATECHOLATE CATALYSTS**
ALKOXYLIERUNGSVERFAHREN UNTER VERWENDUNG VON PHOSPHONIUM-DICATECHOLAT-KATALYSATOREN
PROCÉDÉS D'ALCOXYLATION UTILISANT DES CATALYSEURS À BASE DE DICATÉCHOLATE DE PHOSPHONIUM

(30) Priority: 18.10.2022 US 202263417013 P
(43) Date of publication of application: 09.07.2025
(73) Proprietor: Dow Global Technologies LLC, Midland, MI 48674 (US); Rohm and Haas Company, Collegeville, PA 19426 (US)
(72) Inventor: WALLER, Peter J., Midland, Michigan 48674 (US); BERNALES CANDIA, Sandra Varinia, Midland, Michigan 48667 (US); KENNEDY, Robert, Midland, Michigan 48640 (US); RAGHURAMAN, Arjun, Lake Jacson, Texas 77556 (US); CUMMINS, Clark H., Midland, Michigan 48640 (US); BELOWICH, Matthew E., Collegeville, Pennsylvania 19426 (US)
(74) Representative: Beck Greener LLP
(86) International application number: PCT/US2023/076795
(87) International publication number: WO 2024/086490

(56) References cited:
- US-A1- 2002 183 561
- ROTH DANIEL ET AL: "Lewis Superacidic Catecholato Phosphonium Ions: Phosphorus-Ligand Cooperative C-H Bond Activation", JOURNAL OF THE AMERICAN CHEMICAL SOCIETY, vol. 143, no. 38, 29 September 2021 (2021-09-29), pages 15845 - 15851, XP093119495, ISSN: 0002-7863, DOI: 10.1021/jacs.1c07905

## Description

This invention relates to an alkoxylation process in which a cyclic oxide is added onto a starter compound to produce an ether or polyether.

Polyethers are produced globally in large quantities. Polyether polyols, for example, are important raw materials for producing polyurethanes. Among other things, they are used to make high resiliency, molded, or rigid foams. Polyether monols are used, for example, as surfactants and industrial solvents, among other uses. Carbonate- and ester-modified alkylene oxide polymers also find uses in these and other applications.

Polyether monols and polyols are produced via alkoxylation of a starter compound, in which an active site on the starter reacts with a cyclic oxide in a ring-opening reaction. A terminal hydroxyl group is produced, which in turn can function as an active site for a subsequent alkoxylation step, thereby producing a polyether chain. The active site of the starter compound is a group containing an active hydrogen, such as a hydroxyl or thiol group. The main functions of the starter compound are to provide molecular weight control and to establish the number of hydroxyl groups the alkoxylated product will have.

A catalyst is needed to obtain economical polymerization rates. The most commonly used catalysts are alkali metal hydroxides such as potassium hydroxide and the so-called double metal cyanide (DMC) catalyst complexes, of which zinc hexacyanocobaltate catalyst complexes are the most commercially important type.

Alkali metal hydroxides provide the benefits of low catalyst costs and acceptable alkoxylation rates. They are versatile in that they effectively polymerize many alkylene oxides. Nonetheless, alkali metal hydroxides have well-known drawbacks. The alkoxylated product must be neutralized and catalyst residues scrupulously removed. These finishing steps add greatly to both capital and operating costs, and produce additional waste streams that must be cleaned up and/or disposed of.

DMC catalysts provide rapid polymerization rates compared to alkali metal catalysts, even when used at very low catalyst concentrations. An important advantage of DMC catalysts over alkali metal hydroxides is no neutralization step is needed. The catalyst residues often can be left in the product, unlike the case when alkali metal hydroxides are used as the polymerization catalyst. This can result in significantly lower production costs. Nonetheless, the DMC catalysts have significant disadvantages as well. They tend to perform poorly in the presence of high concentrations of hydroxyl groups, and especially in the presence of low molecular weight starter compounds like glycerol or sorbitol that have hydroxyl groups in the 1,2- or 1,3- positions with respect to each other. Under these conditions, the catalysts are difficult to activate, perform sluggishly and often deactivate before the polymerization is completed. This represents a significant limitation on the widespread adoption of DMC catalysts. It is often necessary to produce the polyether in two or more discrete steps, in which the early stages of the polymerization are conducted in the presence of an alkali metal catalyst and, after cleaning up the resulting intermediate product, the remainder of the polymerization is performed using the DMC catalyst. This approach requires the intermediate to be neutralized and purified (because the DMC catalyst is deactivated by strong bases), thus re-introducing costs which the DMC-catalyzed polymerization is intended to avoid.

Certain Lewis acids have been evaluated as alkylene oxide polymerization catalysts. The Lewis acids require essentially no activation time, but deactivate rapidly and therefore cannot produce high molecular weight polymers or high conversions of alkylene oxide to polymer. Another problem with many Lewis acid catalysts is that they deactivate at higher operating temperatures. This disqualifies them for use with certain starters that are solids, viscous, or otherwise poorly miscible with the cyclic oxide, because in those cases high operating temperatures are needed to melt the starter, reduce its viscosity or promote mixing with the cyclic oxide.

US2002/183561 discloses an alkoxylation process comprising reacting a cyclic oxide with a starter compound in the presence of a base and a phosphonium salt.

Various phosphonium compounds have been described in the literature. *See,* for example, Science 341 1374 (2013), Dalton Trans. 2018, 47, 11411, Chem. Eur. J. 2015, 21, 6491-6500, Dalton Trans. 2016, 45, 5568, Angew. Chem. Int. Ed. 2014, 53, 6538-6541, Chem. Sci. 2015, 6, 2016 and Chem. Commun., 2018, 54, 662-665. Phosphonium dicatecholates are described in J. Am. Chem. Soc. 2021, 143, 15845-15851. These phosphonium compounds have been described for use as catalysts in various reactions such as olefin isomerization, hydrosilylation, dehydrocoupling, hydrodefluorination, hydrogenation and Friedel-Crafts reactions.

This invention is an alkoxylation process comprising (step I) forming a reaction mixture comprising a) a starter compound having at least one hydroxyl or thiol group; b) at least one cyclic oxide and c) a catalytically effective amount of a phosphonium catalyst having either of the structures: 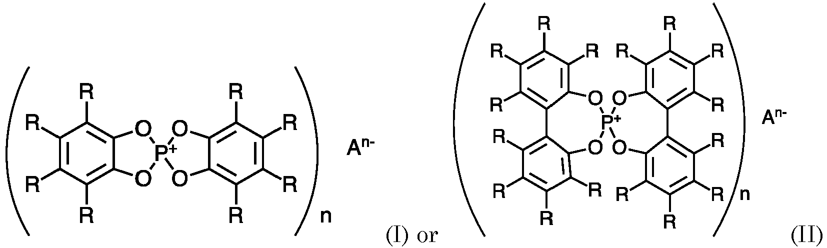 wherein each R is independently hydrogen, halogen, unsubstituted or inertly substituted C₁₋₁₂ alkoxyl, aryloxy, unsubstituted or inertly substituted linear, branched and/or cyclic alkyl and unsubstituted or inertly substituted aryl, and wherein any two R groups may together form a ring structure, A represents a weakly coordinating anion and n represents the valence of A, and (step II) reacting the cyclic oxide with the starter compound in the presence of the phosphonium dicatecholate catalyst to form an alkoxylated starter product.

An advantage of the process of the invention is very high alkoxylation rates are obtained using very small amounts of the phosphonium catalyst. For that reason catalyst residues can be left in the product (unlike potassium hydroxide), thereby reducing or even eliminating catalyst deactivation and removal steps. Unlike DMC catalysts, these compounds are highly effective at polymerizing oxiranes onto very low molecular weight onto a starter compound. The phosphonium catalysts described herein are particularly useful for alkoxylating low molecular weight starters with 1 to 12 oxyalkylene units per active site.

Inert substituents do not react with the starter or cyclic oxide under the conditions of the alkoxylation reaction and include, for example, alkyl (linear, branched and/or cyclic), aryl, ether (-O-), ester (-O-C(O)-), carbonate (-O-C(O)-O))-, halogen (especially F, Cl, Br and/or I), sulfide (-S-), polysulfide (-S_{z}-, where z >1), amino, silyl and the like. The R groups preferably do not contain active sites such as -OH, -NH, -SH or -COOH where alkoxylation can take place, and preferably do not contain cyclic oxide structures.

Examples of R groups in structure I and structure II include hydrogen, F, Cl, Br and I. Other examples of R groups include -O-R¹ wherein R¹ is unsubstituted or inertly substituted linear, branched and/or cyclic alkyl having 1 to 12 carbon atoms (especially 1-4 carbon atoms such as methyl, ethyl, n-propyl, isopropyl, n-butyl, sec-butyl and t-butyl). If R¹ is substituted, a preferred substituent is halogen, especially F or Cl. Specific examples of halogen-substituted R¹ groups include -CF₃, -CCl₃, perfluoroethyl, perchloroethyl, monochloromethyl, monofluoromethyl and the like.

Still other examples of R groups include -O-Ar where Ar designates unsubstituted or inertly substituted aryl (especially phenyl). In embodiments in which Ar is substituted, preferred substituents include halogen, especially F and Cl, lower alkyl such as methyl, ethyl, n-propyl, isopropyl, n-butyl, sec-butyl and t-butyl, and halogen-substituted alkyl such as -CF₃, -CCl₃, perfluoroethyl, perchloroethyl, monochloromethyl, monofluoromethyl and the like.

In embodiments in which two or more R groups together form a ring structure (together with the carbon atoms of the phenyl group to which they are attached), the ring structure may be aliphatic or aromatic and may contain heteroatoms in the ring. When such a ring structure formed by two R groups is aromatic, the ring structure may be fused to the associated phenyl group.

The anion A is a weakly coordinating anion that has a valence of n. n is preferably 1 or 2 and most preferably 1. Weakly coordinating anions are those whose coordination to the associated cation is weaker than that of the surrounding solvent molecules. Coordination strength of an anion is conveniently determined by forming a tri-n-octylammonium salt of the anion, dissolving the salt in carbon tetrachloride, and measuring the N-H stretching frequency by infrared spectroscopy, using a method as described, for example, in J. Am. Chem Soc. 2006, 128, 8500-8508. An N-H stretching frequency of 3000 cm⁻¹ or greater, especially 3050 cm⁻¹ or greater, is indicative of a weakly coordinating anion.

Examples of weakly coordinating anions include tetrakis[perfluorophenyl] borate, tetrakis[3,5-bis(trifluoromethyl)phenyl]borate, trifluoromethanesulfonate (triflate), Al[OC(CF₃)₃]₄⁻, HCB₁₁Me₅F₆⁻, B₁₂F₁₂²⁻, HCB₁₁H₅F₆⁻, B(OTeF₅)₄⁻, Sb(OTeF₅)₆⁻, Al[OC(CF₃)₃]₄⁻, Al[OCH(CF₃)₂]₄⁻ and Al[OC(CH₃)(CF₃)₂]₄⁻.

Specific examples of phosphonium catalysts include: 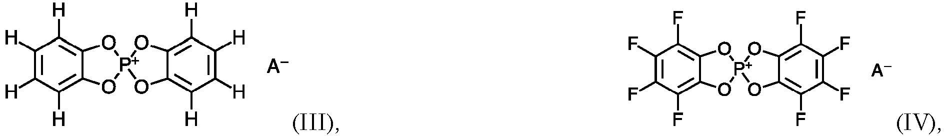 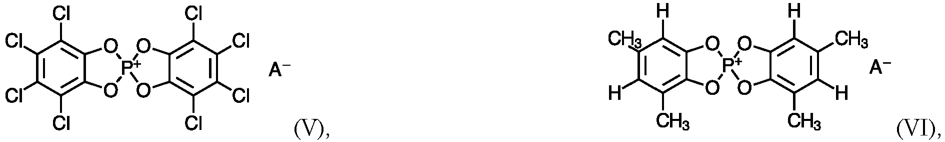 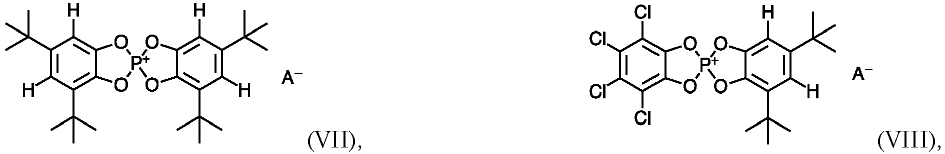 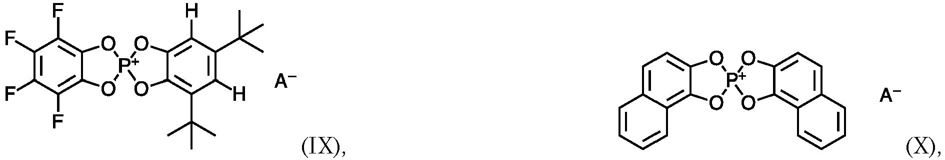 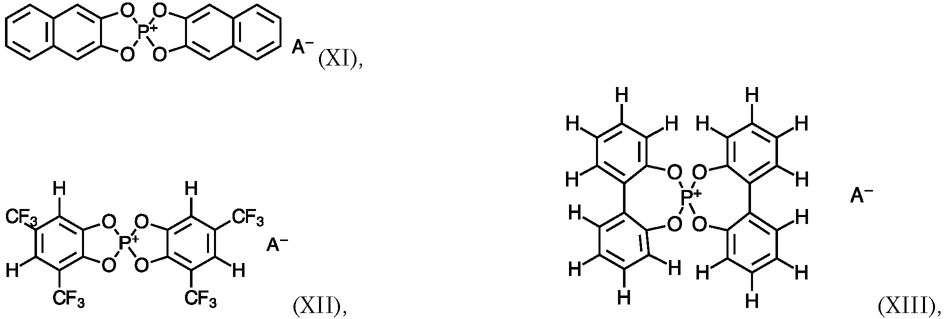 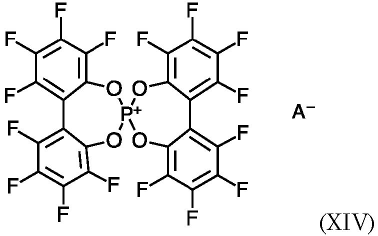 and the like, where in each case A is monovalent. Analogous compounds of the form Z⁺₂A²⁻, where Z⁺ represents the phosphonium cation as shown in any of structures III-XIV and A²⁻ represents a divalent weakly coordinating anion, are also useful.

In any of the foregoing, A⁻ and A²⁻ may be any of the weakly coordinating anions mentioned before, in particular a monovalent anion such as tetrakis(perfluorophenyl)borate, tetrakis[3,5-bis(trifluoromethyl)phenyl]borate and trifluoromethanesulfonate (triflate).

Methods useful for preparing the phosphonium catalyst of structure I are generally described *in* J. Am. Chem. Soc. 2021, 143, 15845-15851.

In general, symmetric phosphonium dicatecholate catalysts can be prepared by reacting catechol or a catechol derivative having the structure 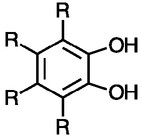 with PCl₅ in solution in dichloromethane or other suitable solvent to form the corresponding chlorophosphorane 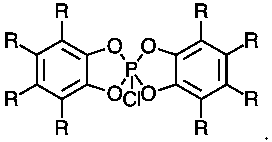 Reaction with a sodium or other suitable salt of the A anion (such as sodium tetrakis(pentafluorophenyl)borate) produces the phosphonium catalyst.

Asymmetric phosphonium catalysts of structure I can be prepared by reacting catechol or a catechol derivative having the structure 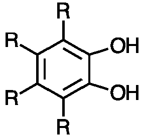 with PCl₅ in solution in dichloromethane or other suitable solvent to produce 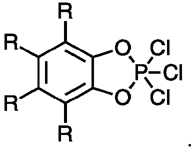 This compound is further reacted with a second but different catechol or catechol derivative having the structure 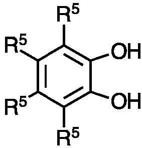 wherein each R⁵ as defined the same way as R, to produce the chlorophosphorane 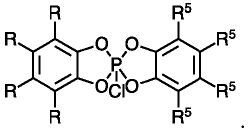 The chlorophosphorane is converted to the phosphonium catalyst by reaction with a sodium or other suitable salt of the A anion (such as sodium tetrakis(pentafluorophenyl)borate).

Compounds of the form of Structure II can be synthesized analogously: an appropriately-substituted biphenol is reacted with PCl₅, giving an intermediate phosphorus chloride, from which salt metathesis with an alkali tetrakis(perfluorophenyl)borate provides the desired product.

The alkoxylation is performed in the presence of one or more starter compounds. The starter compound has one or more functional groups capable of being alkoxylated. The starter may contain any larger number of such functional groups. The functional groups may be, for example, primary, secondary or tertiary hydroxyl, or thiol. A preferred starter contains 1 or more such functional groups, preferably 2 or more of such functional groups, and may contain as many as 12 or more of such functional groups.

In certain embodiments, the functional groups are all hydroxyl groups. In some embodiments, the starter compound will have 2 to 8, 2 to 6, 2 to 4 or 2 to 3 hydroxyl groups.

The starter compound has an equivalent weight per functional group less than that of the polyether product. It may have an equivalent weight of 9 g/equivalent (in the case of water) to 6000 g/equivalent or more. The invention has particular advantages when the starter compound is a low equivalent weight alcohol or polyol (up to 500, up to 250, up to 125 and especially up to 80 g/equivalent, for example) and for that reason prior to alkoxylation has a high concentration of hydroxyl groups. Equivalent weight of an alcohol or polyol is conveniently determined using titration methods such as ASTM 4274-16, which yield a hydroxyl number in mg KOH/gram of polyol that can be converted to equivalent weight using the relation equivalent weight = 56,100 ÷ hydroxyl number.

Among the suitable starters are vinyl alcohol, propenyl alcohol, allyl alcohol, acrylic acid, hydroxyethyl acrylate, hydroxyethyl methacrylate, a C₁₋₅₀ alkanol, cyclohexanol, water, ethylene glycol, diethylene glycol, triethylene glycol, propylene glycol, dipropylene glycol, tripropylene glycol, 1,4-butane diol, 1,6-hexane diol, 1,8-octane diol, cyclohexane dimethanol, glycerol, trimethylolpropane, trimethylolethane, pentaerythritol, sorbitol, xylitol, mannitol, maltitol, sucralose, phenol, an alkylphenol, polyphenolic starters such as bisphenol-A and 1,1,1-tris(hydroxyphenyl)ethane, and the like. Any two or more of the foregoing starters may be used together if desired.

The cyclic oxide is characterized in having a least one 3-, 4- or 5- member ring structure that contains an oxygen atom in the ring structure. Especially preferred cyclic oxides are oxiranes that have a three-member, oxygen containing ring. The cyclic oxide(s) may be, for example, ethylene oxide, 1,2-propylene oxide (generally referred to herein as "propylene oxide"), oxetane, 1,2-butene oxide, 2-methyl-1,2-butene oxide, 2,3-butene oxide, tetrahydrofuran, epichlorohydrin, hexene oxide, octene oxide, styrene oxide, divinylbenzene dioxide, a glycidyl ether such as bisphenol-A diglycidyl ether, epichlorohydrin or other polymerizable oxirane. In some embodiments, the alkylene oxide is propylene oxide, ethylene oxide, or a mixture thereof, including, for example, a mixture of at least 50% (preferably at least 80%) by weight propylene oxide and correspondingly up to 50% (preferably up to 20%) by weight ethylene oxide. In some embodiments, two or more alkylene oxides are polymerized simultaneously (to form random copolymers), and or the composition of the alkylene oxide is changed one or more times, or even continuously, throughout the course of the polymerization to form block and/or random/block copolymers.

The alkoxylation is performed by combining the starter and phosphonium catalyst with the cyclic oxide(s) and optionally comonomer and subjecting the resulting reaction mixture to reaction conditions. The catalyst may be added as a solution in a solvent. Such a solvent preferably is inert under the conditions of the alkoxylation reaction. Dichloromethane and dichloroethane are useful solvents for the phosphonium catalyst.

The alkoxylation proceeds at a wide range of temperatures from -100°C to 250°C or more. In some embodiments, the reaction temperature is at least 80°C, at least 100°C, at least 120°C, at least 130°C or at least 150°C. The polymerization temperature preferably does not exceed 190°C, and more preferably does not exceed 180°C. An important advantage of the phosphonium catalysts used in the invention is they perform well without premature deactivation at higher temperatures, especially 150° to 200°C or 150° to 180°C. The higher temperatures promote faster reactions. Additionally, the ability to operate at these higher temperatures permits the process to be used with starters and/or cyclic oxides that have somewhat high melting temperatures (such as sorbitol, xylitol, mannitol, maltitol and sucralose) and/or which are viscous at lower temperatures, or which, like sorbitol and glycerol, have limited solubility in the cyclic oxide at lower temperatures.

The alkoxylation reaction usually is performed at a super atmospheric pressure, but it can be performed at atmospheric pressure or even a subatmospheric pressure.

Enough phosphonium catalyst is used to provide a commercially reasonable alkoxylation rate, but it is generally desirable to use as little thereof as possible consistent with reasonable alkoxylation rates, as this both reduces the cost for the catalyst and can eliminate the need to remove catalyst residues from the product. The amount of phosphonium catalyst may be, for example, sufficient to provide 10 to 10,000 ppm by weight of phosphonium catalyst based on the weight of the starter. In specific embodiments, the amount of phosphonium catalyst may be sufficient to provide at least 25 ppm, at least 50 ppm or at least 100 ppm catalyst on the foregoing basis, and up to 1,000 ppm or up to 500 ppm catalyst, again on the foregoing basis. The weight of the phosphonium catalyst includes the weight of both cation and associated anion.

The alkoxylation reaction can be performed batch-wise, semi-continuously (including with continuous addition of starter as described in US 5,777,177) or continuously.

The alkoxylation reaction can be performed in any type of vessel that is suitable for the pressures and temperatures encountered. The reactor should be equipped with a means of providing and/or removing heat, so the temperature of the reaction mixture can be maintained within the required range. Suitable means include various types of jacketing for thermal fluids, various types of internal or external heaters, and the like. A cook-down step performed on continuously withdrawn product is conveniently conducted in a reactor that prevents significant back-mixing from occurring. Plug flow operation in a pipe or tubular reactor is a preferred manner of performing such a cook-down step.

The crude product obtained in any of the foregoing processes may contain unreacted cyclic oxide, small quantities of the starter compound and low molecular weight alkoxylates thereof, and small quantities of other organic impurities and/or water. Volatile impurities (including unreacted cyclic oxides) should be flashed or stripped from the product. The crude product typically contains catalyst residues. It is typical to leave these residues in the product, but these can be removed if desired. Moisture and volatiles can be removed by stripping the alkoxylated product.

The process of the invention is useful for preparing alkoxylated products that can have hydroxyl equivalent weights from as low as about 85 g/equivalent to as high as about 8,000 g/equivalent or more. Alkoxylated polyols produced in accordance with the invention are useful raw materials for producing polyurethanes and other polymers made by reacting the alkoxylated polyol with a polyisocyanate. These products include a wide variety of cellular and non-cellular materials, which may vary in physical properties from very rigid to highly flexible. Alkoxylated monols produced in accordance with the invention are useful as surfactants or as industrial solvents, among other uses. Alkoxylated polyols and monols can be aminated to produce the corresponding amine-terminated materials, which are in turn useful raw materials for making various materials including polyureas and cured epoxy resins.

In particular embodiments, the starter is a polyol having a hydroxyl equivalent weight of 125 g/equivalent or less, especially 75 g/equivalent or less or even 50 g/equivalent or less, and the alkoxylation is continued to produce an alkoxylated product having 1 to 12, especially 1 to 10, 1 or 5 or 1 to 3 units of polymerized cyclic oxide per hydroxyl group on the starter. The number average molecular weight of the alkoxylated product may be, for example, 100 to 1000 g/mol, 100 to 8500 g/mol, 150 to 800 g/mol or 200 to 800 g/mol. In such particular embodiments, the cyclic oxide is preferably 1,2-propylene oxide, ethylene oxide, 1,2-butylene oxide, 2,3-butylene oxide, epichlorohydrin or a mixture of any two or more thereof, with 1,2-propylene oxide, ethylene oxide or a mixture thereof being particularly preferred. The starter in such embodiments most preferably is one or more of glycerol, trimethylolpropane, trimethylolethane, erythritol, pentaerythritol, sorbitol and sucrose. Such products are useful as raw materials for making rigid polyurethane and/or polyisocyanurate polymers, including foams.

In some embodiments the cyclic oxide is polymerized with or in the presence of one or more copolymerizable monomers that are not cyclic oxides. Examples of such copolymerizable monomers include carbonate precursors that copolymerize with an alkylene oxide to produce carbonate linkages in the product. Examples of such carbonate precursors include carbon dioxide, phosgene, linear carbonates and cyclic carbonates. Other copolymerizable monomers include carboxylic acid anhydrides, which copolymerize with cyclic oxides to produce ester linkages in the product.

The following examples are provided to illustrate the invention but are not intended to limit the scope thereof. All parts and percentages are by weight unless otherwise indicated.

### Examples 1-2 and Comparative Samples A-C

The tetrakis(pentafluorophenyl) borate salt of phosphonium dicatecholate, 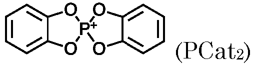 is made according to the method described in J. Am. Chem. Soc. 2021, 143, 15845-15851.

45 grams of starter as indicated in Table 1 are charged into a semi-batch reactor equipped with stirrer, temperature controls, nitrogen feed and monomer feed lines and a vent. Catalyst is added as a solid to the starter. The type and amount of catalyst (based on starter) are as indicated in Table 1. The reactor is purged with nitrogen and heated to the temperature indicated in Table 1 with stirring, then purged again with nitrogen to remove any solvent from the catalyst addition. While maintaining the same temperature, propylene oxide then is fed into the reactor on demand to attempt to maintain a target propylene oxide partial pressure as indicated in Table 1. The target amount of propylene oxide to be added is approximately 103 g in the case of polyols initiated from glycerol, and 150 g in the case of polyols initiated from sorbitol; the actual amounts fed are indicated in Table 1. The time required to feed the propylene oxide (run time) is indicated in Table 1. Upon completion of monomer feed, the reaction is digested at 160°C for 2 hours and then cooled to 50°C under nitrogen purge. After purging with nitrogen at 50°C for 10 minutes, the product is collected and yield is calculated. The product is analyzed for Mₙ and polydispersity by gel permeation chromatography against polystyrene standards.

The activities of the catalysts are compared by calculating a turnover frequency (TOF) in each instance. TOF reflects the number of propylene oxide molecules converted per catalytic site per unit time, as follows: $TOF = \frac{mmol PO consumed}{mmol catalyst \times run time \left(hr\right)} .$ Higher values indicate greater catalyst activity.

In Table 1, KOH designates potassium hydroxide and BF₃·OEt₂ designates boron trifluoride diethyl etherate.

**Table 1**

| Designation | Catalyst | Loading (ppm) | T (°C) | Start -er | PO partial pressure, psi (kPa) | Run time (h) | PO Fed (mL) | Yield (g) | TOF (hr⁻¹) | Mₙ, g /mol | PDI |
|---|---|---|---|---|---|---|---|---|---|---|---|
| A* | KOH | 4000 | 130 | Gly | 30 (207) | 1.6 | 103.8 | 112.3 | 207 | 412 | 1.02 |
| B* | BF₃.O Et₂ | 111 | 100 | Gly | 11 (76) | 47.2 | 24.5 | 37.1 | 211 | ND | ND |
| C* | B(C₆F ₅)₃ | 667 | 80 | Gly | 7 (48) | 1.7 | 103.0 | 114.7 | 14,768 | 407 | 1.10 |
| 1 | PCat₂ | 378 | 160 | Gly | 30 (207) | 0.6 | 103.1 | 114.2 | 138,321 | 386 | 1.11 |
| 2 | PCat₂ | 389 | 160 | Sorb | 30 (207) | 1.1 | 154.8 | 136.6 | 109,505 | 764 | 1.11 |

| | | | | | | | | | | | |
|---|---|---|---|---|---|---|---|---|---|---|---|
| *Not an example of the invention. 'ND" is not done. "Gly" is glycerol. "Sorb" is sorbitol. "PO partial pressure" is the target PO partial pressure in the reactor during the polymerization. The "Run time" indicates the time required to feed the indicated amount of propylene oxide. "PO Fed" indicates the total amount of propylene oxide fed during the indicated run time. "TOF" is turnover frequency. PDI is the polydispersity index, i.e., weight average molecular weight divided by number average molecular weight. Molecular weights are measured by GPC against polystyrene standards. | | | | | | | | | | | |

As indicated by the data in Table 1, the catalyst of the invention is extremely active compared to the controls. Turnover frequencies range are approximately 500 to 650 times greater than that of KOH, which is the industry workhorse propylene oxide polymerization catalyst. The greater catalytic activity leads to drastically reduced run times, effectively increasing the production capability of the manufacturing equipment proportionally. Molecular weight and polydispersity are similar to those obtained in the KOH-catalyzed run (Comp. A).

### Parallel Pressure Reactor (PPR) Polymerization Procedure

Ethylene oxide polymerizations are performed on using a 48-well Symyx Technologies Parallel Pressure Reactor (PPR). Each of the 48 wells is equipped with an individually weighed glass insert having an internal working liquid volume of approximately 5 mL. The wells each contain an overhead paddle stirrer.

0.7 mL of a glycerol/PCat₂ mixture (containing approximately 0.72 g of the starter) is charged into an insert. This mixture provides about 500 ppm by weight of catalyst based on the combined weight of starter and ethylene oxide used in the polymerization run. The well is pressurized with 50 psig (344.7 kPa) of nitrogen and then heated to the polymerization temperature of 160°C. Upon reaching the polymerization temperature, 0.67 mL of ethylene oxide is injected into the well, where it reacts with the starter in the glass insert.

Another 0.67 mL of the ethylene oxide is injected an hour after the start of polymerization, and again after the second hour of polymerization. 4 hours after the first ethylene oxide injection, the well is allowed to cool to room temperature and vented. The glass insert is allowed to stand under nitrogen at 40-50°C overnight to allow residual ethylene oxide to volatilize, after which the insert is weighed to determine the amount of product.

The resulting product is analyzed for molecular weight and polydispersity (M_{w}/Mₙ) by gel permeation chromatography against a polystyrene standard.

Polymerization at 160°C results in 82% conversion of ethylene oxide to polymer. The number average molecular weight of the product is 280 and polydispersity is 1.01.

## Claims

1. An alkoxylation process, comprising:
(step I) forming a reaction mixture comprising;
a) a starter compound having at least one hydroxyl or thiol group,
b) at least one cyclic oxide, and
c) a catalytically effective amount of a phosphonium dicatecholate catalyst having the structure: 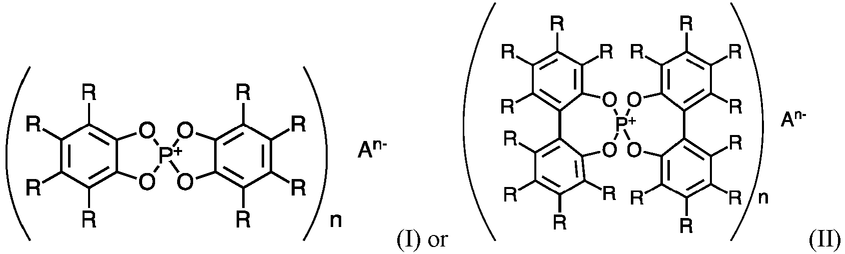
wherein each R is independently hydrogen, halogen, unsubstituted or inertly substituted C₁₋₁₂ alkoxyl, aryloxy, unsubstituted or inertly substituted linear, branched and/or cyclic alkyl and unsubstituted or inertly substituted aryl, wherein any two R groups may together form a ring structure, A represents a weakly coordinating anion and n represents the valence of A; and
(step II) reacting the cyclic oxide b) with the starter compound a) in the presence of the phosphonium dicatecholate catalyst c) to form an alkoxylated starter product.

2. The alkoxylation process of claim 1, wherein the phosphonium dicatecholate catalyst c) has any one of the structures: 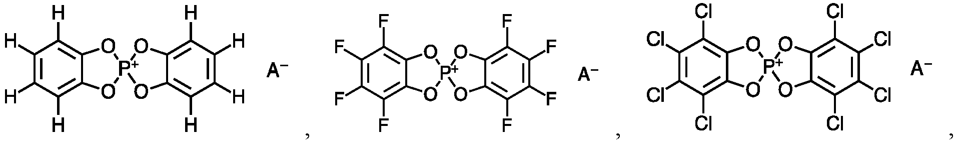 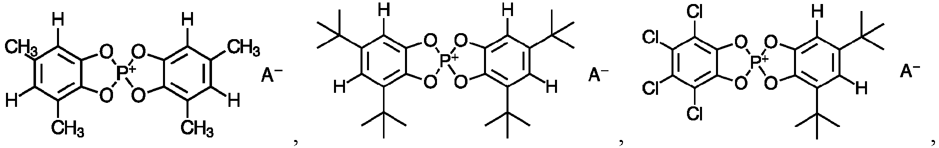 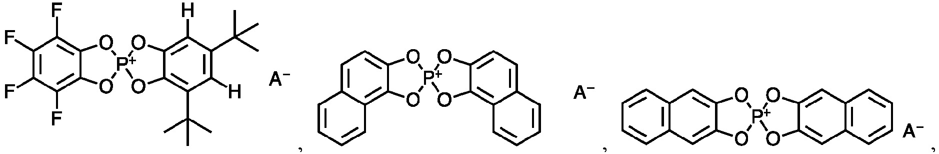 and 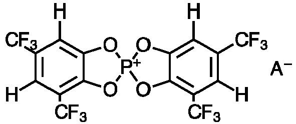 and where A⁻ is a weakly coordinating anion.

3. The alkoxylation process of claim 1 or 2, wherein A is selected from the group consisting of tetrakis[perfluorophenyl] borate, tetrakis[3,5-bis(trifluoromethyl)phenyl]borate, trifluoromethanesulfonate, Al[OC(CF₃)₃]₄⁻, HCB₁₁Me₅F₆⁻, HCB₁₁H₅F₆⁻, B(OTeF₅)₄⁻, Sb(OTeF₅)₆⁻, Al[OC(CF₃)₃]₄⁻, Al[OCH(CF₃)₂]₄⁻ and Al[OC(CH₃)(CF₃)₂]₄⁻.

4. The alkoxylation process of any one preceding claim, wherein the phosphonium dicatecholate catalyst is 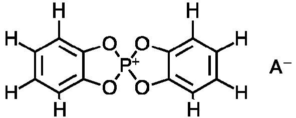 and where A⁻ is a weakly coordinating anion.

5. The alkoxylation process of claim 4, wherein A is tetrakis(perfluorophenyl)borate, tetrakis[3,5-bis(trifluoromethyl)phenyl]borate or trifluoromethanesulfonate.

6. The alkoxylation process of claim 1, wherein A is B₁₂F₁₂²⁻ and n is 2.

7. The alkoxylation process of any one preceding claim, wherein the starter compound a) has an equivalent weight of 80 g/equivalent or less.

8. The alkoxylation process of any one preceding claim, wherein the starter compound a) is one or more of vinyl alcohol, propenyl alcohol, allyl alcohol, acrylic acid, hydroxyethyl acrylate, hydroxyethyl methacrylate, a C₁₋₅₀ alkanol, cyclohexanol, water, ethylene glycol, diethylene glycol, triethylene glycol, propylene glycol, dipropylene glycol, tripropylene glycol, 1,4-butane diol, 1,6-hexane diol, 1,8-octane diol, cyclohexane dimethanol, glycerol, trimethylolpropane, trimethylolethane, pentaerythritol, sorbitol, sucrose, xylitol, mannitol, maltitol, sucralose, phenol, an alkylphenol, bisphenol-A and 1,1,1-tris(hydroxyphenyl)ethane.

9. The alkoxylation process of any one preceding claim, wherein the cyclic oxide b) is an oxirane.

10. The alkoxylation process of claim 9, wherein the cyclic oxide b) is one or more of ethylene oxide, 1,2-propylene oxide, 1,2-butene oxide and 2,3-butene oxide.

11. The alkoxylation process of any one preceding claim, wherein step II is performed at a temperature of 150 to 200°C.

## Patentansprüche

1. Alkoxylierungsprozess, umfassend:
(Schritt I) Ausbilden eines Reaktionsgemisches, umfassend;
a) eine Starterverbindung, die mindestens eine Hydroxyl- oder Thiolgruppe aufweist,
b) mindestens ein zyklisches Oxid, und
c) eine katalytisch wirksame Menge eines Phosphoniumdicatecholat-Katalysators, der die Struktur aufweist: 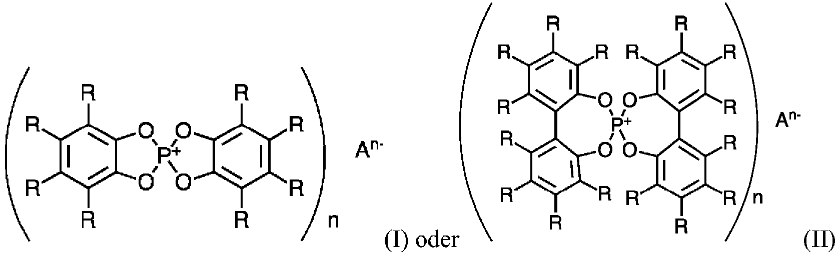
wobei jedes R unabhängig Wasserstoff, Halogen, unsubstituiertes oder inert substituiertes C₁₋₁₂-Alkoxyl, Aryloxy, unsubstituiertes oder inert substituiertes lineares, verzweigtes und/oder zyklisches Alkyl und unsubstituiertes oder inert substituiertes Aryl ist, wobei zwei beliebige R-Gruppen zusammen eine RingStruktur ausbilden können, A ein schwach koordinierendes Anion darstellt und n die Valenz von A darstellt; und
(Schritt II) Reagierenlassen des zyklischen Oxids b) mit der Starterverbindung a) in der Gegenwart des Phosphoniumdicatecholat-Katalysators c), um ein alkoxyliertes Starterprodukt auszubilden.

2. Alkoxylierungsprozess nach Anspruch 1, wobei der Phosphoniumdicatecholat-Katalysator c) eine beliebige der Strukturen aufweist: 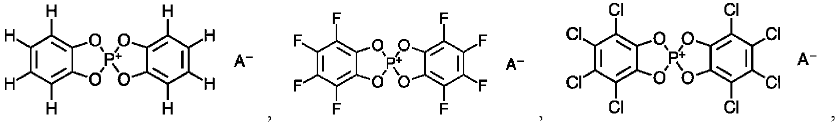 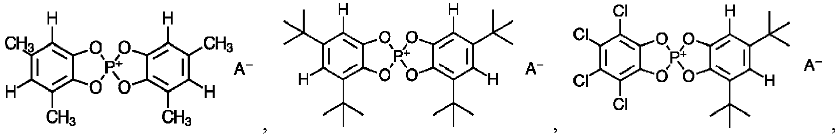 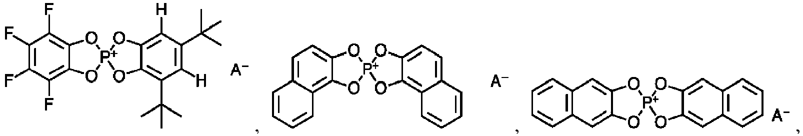 und 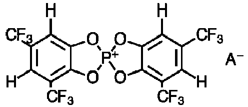 und wobei A- ein schwach koordinierendes Anion ist.

3. Alkoxylierungsprozess nach Anspruch 1 oder 2, wobei A aus der Gruppe ausgewählt ist, bestehend aus Tetrakis[perfluorphenyl]borat, Tetrakis[3,5-bis(trifluormethyl)phenyl]borat, Trifluormethansulfonat, Al[OC(CF₃)₃]₄⁻, HCB₁₁Me₅F₆⁻, HCB₁₁H₅F₆⁻, B(OTeF₅)₄⁻, Sb(OTeF₅)₆⁻, Al[OC(CF₃)₃]₄⁻, Al[OCH(CF₃)₂]₄⁻und Al[OC(CH₃)(CF₃)₂]₄⁻.

4. Alkoxylierungsprozess nach einem der vorstehenden Ansprüche, wobei der Phosphoniumdicatecholat-Katalysator 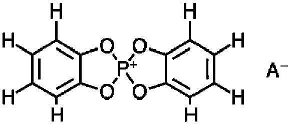 ist und wobei A⁻ ein schwach koordinierendes Anion ist.

5. Alkoxylierungsprozess nach Anspruch 4, wobei A Tetrakis(perfluorphenyl)borat, Tetrakis[3,5-bis(trifluormethyl)phenyl]borat oder Trifluormethansulfonat ist.

6. Alkoxylierungsprozess nach Anspruch 1, wobei A B₁₂F₁₂²⁻ ist und n 2 ist.

7. Alkoxylierungsprozess nach einem vorstehenden Anspruch, wobei die Starterverbindung a) ein Äquivalentgewicht von 80 g/Äquivalent oder weniger aufweist.

8. Alkoxylierungsprozess nach einem der vorstehenden Ansprüche, wobei die Starterverbindung a) eines oder mehrere aus Vinylalkohol, Propenylalkohol, Allylalkohol, Acrylsäure, Hydroxyethylacrylat, Hydroxyethylmethacrylat, einem C₁₋₅₀-Alkanol, Cyclohexanol, Wasser, Ethylenglykol, Diethylenglykol, Triethylenglykol, Propylenglykol, Dipropylenglykol, Tripropylenglykol, 1,4-Butandiol, 1,6-Hexandiol, 1,8-Octandiol, Cyclohexandimethanol, Glycerin, Trimethylolpropan, Trimethylolethan, Pentaerythrit, Sorbit, Saccharose, Xylit, Mannit, Maltit, Sucralose, Phenol, einem Alkylphenol, Bisphenol-A und 1,1,1-tris(Hydroxyphenyl)ethan ist.

9. Alkoxylierungsprozess nach einem der vorstehenden Ansprüche, wobei das zyklische Oxid b) ein Oxiran ist.

10. Alkoxylierungsprozess nach Anspruch 9, wobei das zyklische Oxid b) eines oder mehrere von Ethylenoxid, 1,2-Propylenoxid, 1,2-Butenoxid und 2,3-Butenoxid ist.

11. Alkoxylierungsprozess nach einem der vorstehenden Ansprüche, wobei Schritt II bei einer Temperatur von 150 bis 200 °C durchgeführt wird.

## Revendications

1. Procédé d'alcoxylation, comprenant :
(étape I) la formation d'un mélange réactionnel comprenant ;
a) un composé de départ ayant au moins un groupe hydroxyle ou thiol,
b) au moins un oxyde cyclique, et
c) une quantité efficace catalytiquement d'un catalyseur dicatécholate de phosphonium ayant la structure : 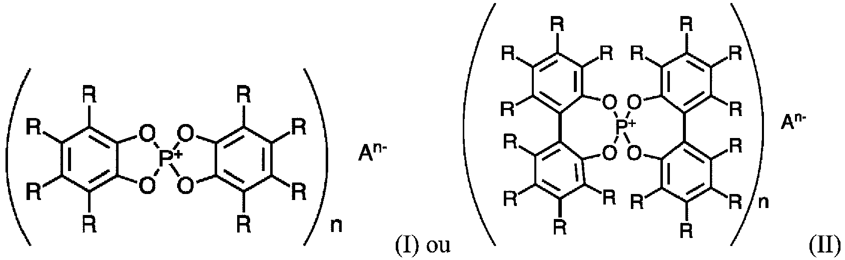
dans lequel chaque R est indépendamment hydrogène, halogène, alcoxyle en C₁₋₁₂ non substitué ou inerte, aryloxy, alkyle et/ou cyclique non substitué ou inerte, ramifié et aryle non substitué ou substitué inerte, dans lequel deux groupes R quelconques peuvent former ensemble une structure cyclique, A représente un anion faiblement coordinateur et n représente la valence de A ; et
(étape II) la réaction de l'oxyde cyclique b) avec le composé de départ a) en présence du catalyseur dicatécholate de phosphonium c) pour former un produit de départ alcoxylé.

2. Procédé d'alcoxylation selon la revendication 1, dans lequel le catalyseur dicatécholate de phosphonium c) a l'une quelconque des structures : 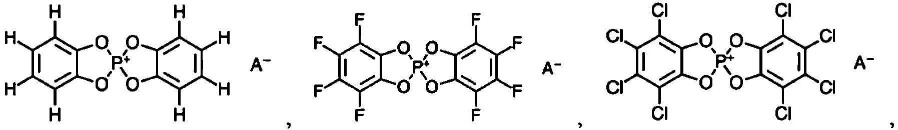 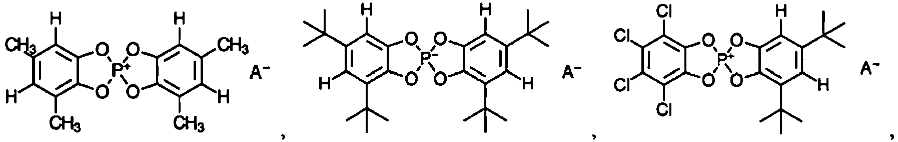 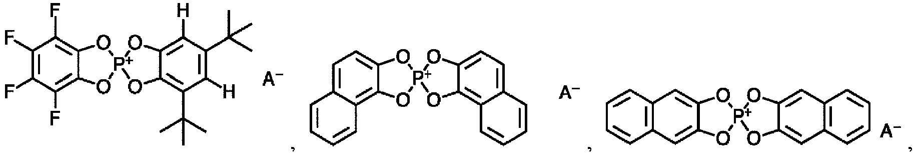 et 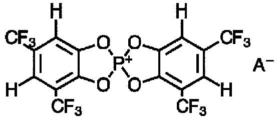 et où A⁻est un anion faiblement coordonnateur.

3. Procédé d'alcoxylation selon la revendication 1 ou 2, dans lequel A est choisi dans le groupe constitué de tétrakis[perfluorophényl]borate, tétrakis[3,5-bis(trifluorométhyl)phényle]borate, trifluorométhanesulfonate, Al[OC(CF₃)₃]₄⁻, HCB₁₁Me₅F₆⁻, HCB₁₁H₅F₆⁻, B(OTeF₅)₄⁻, Sb(OTeF₅)₆⁻, Al[OC(CF₃)₃]₄⁻, Al[OCH(CF₃)₂]₄⁻ et Al[OC(CH₃)(CF₃)₂]₄⁻.

4. Procédé d'alcoxylation selon l'une quelconque revendication précédente, dans lequel le catalyseur dicatécholate de phosphonium est 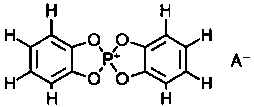 et où A⁻ est un anion faiblement coordinateur.

5. Procédé d'alcoxylation selon la revendication 4, dans lequel A est tétrakis(perfluorophényl)borate, tétrakis[3,5-bis(trifluorométhyl)phényle]borate ou trifluorométhanesulfonate.

6. Procédé d'alcoxylation selon la revendication 1, dans lequel A est B,₁₂F_{, 12}²⁻ et n vaut 2.

7. Procédé d'alcoxylation selon l'une quelconque revendication précédente, dans lequel le composé de départ a) a un poids équivalent de 80 g/équivalent ou moins.

8. Procédé d'alcoxylation selon l'une quelconque revendication précédente, dans lequel le composé de départ a) est un ou plusieurs parmi alcool vinylique, alcool de propényle, alcool d'allyle, acide acrylique, acrylate d'hydroxyéthyle, méthacrylate d'hydroxyéthyle, alcanol en C₁₋₅₀, cyclohexanol, eau, éthylène glycol, diéthylène glycol, triéthylène glycol, propylène glycol, dipropylène glycol, tripropylène glycol, 1,4-butane diol, 1,6-hexane diol, 1,8-octane diol, cyclohexane diméthanol, glycérol, triméthylolpropane, triméthyloléthane, pentaérythritol, sorbitol, saccharose, xylitol, mannitol, maltitol, sucralose, phénol, alkylphénol, bisphénol-A et 1,1,1-tris(hydroxyphényl)éthane.

9. Procédé d'alcoxylation selon l'une quelconque revendication précédente, dans lequel l'oxyde cyclique b) est un oxirane.

10. Procédé d'alcoxylation selon la revendication 9, dans lequel l'oxyde cyclique b) est un ou plusieurs parmi l'oxyde d'éthylène, l'oxyde de 1,2-propylène, l'oxyde de 1,2-butène et l'oxyde de 2,3-butène.

11. Procédé d'alcoxylation selon l'une quelconque revendication précédente, dans lequel l'étape Il est mise en oeuvre à une température de 150 à 200 °C.
